# FASCICULE DE BREVET EUROPEEN

(11) **EP 0 521 779 B1**
(45) Date de publication et mention de la délivrance du brevet: **14.06.1995**
(21) Numéro de dépôt: 92401871.6
(22) Date de dépôt: 01.07.1992
(51) Int. Cl.: C07C 219/32, C07C 255/43, A01N 53/00

(54) **Nouveaux esters pyréthrinoides de l'alcool 4-amino 2,3,5,6-tétrafluorophényl méthylique, leur procédé de préparation et leur application comme pesticides**
Pyrethroide Ester des 4-Amino-2,3,5,6-Tetrafluorphenylmethylalkohols, Verfahren zu ihrer Herstellung und ihre Anwendung als Pestizide
Pyrethroid esters of 4-amino-2,3,5,6-tetraflurophenyl methyl alcohol, process for their preparation and their application as pesticides

(30) Priorité: 04.07.1991 FR 9108378
(43) Date de publication de la demande: 07.01.1993
(73) Titulaire: ROUSSEL-UCLAF, 75007 Paris (FR)
(72) Inventeur: Benoit, Marc, F-13360 Roquevaire (FR); Demassey, Jacques, F-77144 Montevrain (FR); Demoute, Jean-Pierre, F-93360 Neuilly Plaisance (FR)
(74) Mandataire: Tonnellier, Marie-José

(56) Documents cités:
- EP-A- 0 031 199
- EP-A- 0 041 021
- EP-A- 0 281 439

## Description

La présente invention concerne de nouveaux esters pyréthrinoïdes de l'alcool 4-amino 2,3,5,6-tétrafluorophényl méthylique, leur procédé de préparation et leur application comme pesticides.

On connaissait déjà des esters pyréthrinoïdes de l'alcool 4Y 2,3,5,6 tetrafluorophenulmethylique présentant des propriétés pesticides (cf EP281429).

On connaissait également des esters pyréthrinoïdes de l'alcool 4-amino 2,3,5,6-tetrafluorobenzylique présentant des propriétés pesticides (cf EP31199).

L'invention a pour objet les composés de formule (I) : 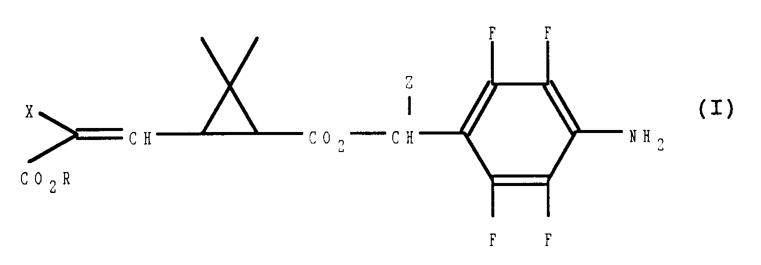
dans lesquels :
- X représente un atome d'hydrogène, de fluor, de chlore ou de brome,
- R représente un radical alkyle linéaire ramifié ou cyclique, saturé ou insaturé renfermant jusqu'à 8 atomes de carbone éventuellement substitué, un radical aryle renfermant jusqu'à 14 atomes de carbone éventuellement substitué ou un radical hétérocyclique éventuellement substitué,
- Z représente un atome d'hydrogène, un radical CH₃, C≡N ou C≡CH, sous toutes leurs formes stéréoisomères possibles ainsi que leurs mélanges.

Lorsque R représente un radical alkyle saturé, linéaire ou ramifié il s'agit de préférence d'un radical méthyle, éthyle, propyle, isopropyle, butyle, sec-butyle, isobutyle, pentyle, hexyle, tert-butyle, tert-pentyle ou néopentyle.

Lorsque R représente un radical cyclique, il s'agit de préférence d'un radical cyclopropyle, cyclobutyle, cyclopentyle ou cyclohexyle, d'un radical alkyle, linéaire ou ramifié, portant un radical cyclique ou d'un radical cyclopropyle, cyclobutyle, cyclopentyle ou cyclohexyle substitué par un ou plusieurs radicaux alkyles dont la liaison avec le groupement -COO- est située sur l'un quelconque de ses sommets, par exemple, le radical 1-méthylcyclobutyle, 1-méthylcyclopentyle, 1-méthylcyclohexyle ou 2,2,3,3-tétraméthylcyclopropyle.

Lorsque R représente un radical alkyle insaturé, il s'agit d'un radical éthylénique, comme par exemple d'un radical vinyle ou 1,1-diméthylallyle ou d'un radical acétylénique, comme, par exemple, le radical éthynyle ou propynyle.

Lorsque R représente un radical alkyle substitué par un ou plusieurs groupements fonctionnels, on entend de préférence par alkyle un radical renfermant de 1 à 8 atomes de carbone, comme, par exemple, le radical méthyle, éthyle, propyle, isopropyle, butyle, isobutyle ou tert-butyle.

Lorsque R représente un radical alkyle substitué par un ou plusieurs groupements fonctionnels, on entend de préférence par groupement fonctionnel, un atome d'halogène, un groupement OR' ou SR' dans lesquels R' représente un atome d'hydrogène ou un radical alkyle renfermant de 1 à 8 atomes de carbone, un groupement : 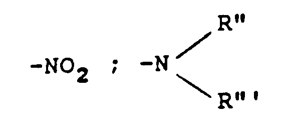
dans lequel R'' et R''', identiques ou différents, représentent un atome d'hydrogène, ou un radical alkyle renfermant de 1 à 8 atomes de carbone, un groupement C≡N, SO₃H ou PO₄H₂ ou un groupement -COalc₁, SO₂alc₂, ou SO₃alc₃ dans lesquels alc₁, alc₂ et alc₃ représentent des radicaux alkyles renfermant de 1 à 18 atomes de carbone.

R peut représenter également un radical alkyle substitué par un radical aryle comme, par exemple, le radical benzyle ou le radical phénéthyle, lui-même éventuellement substitué par un ou plusieurs groupements -OH, -Oalc ou alc renfermant de 1 à 8 atomes de carbone, par un ou plusieurs halogènes ou groupement -CF₃ -OCF₃, -SCF₃ ou par un groupement (G) : 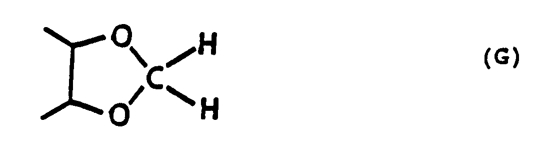
R peut représenter également un radical alkyle substitué sur deux carbones adjacents par un groupement (G₁) : 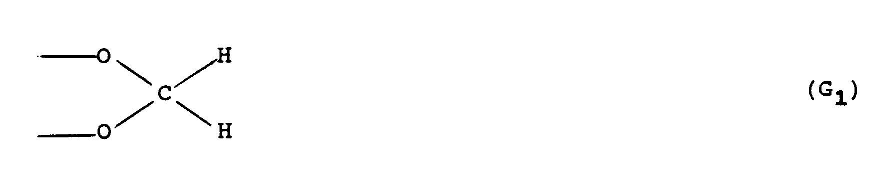
substitué par un groupement : 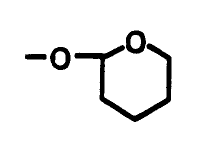
Lorsque R représente un radical alkyle substitué par un ou plusieurs groupements fonctionnels on peut citer comme valeurs préférées de R les radicaux :
-(CH₂)ₙ-C(Hal)₃ dans lequel n est un entier de 1 à 8 et Hal un atome d'halogène, par exemple le radical -CH₂CCl₃, -CH₂CF₃, -CH₂-CH₂-CCl₃ ou -CH₂-CH₂-CF₃,
-(CH₂)ₙ₁-CH(Hal)₂ dans lequel Hal est défini comme ci-dessus et n₁ est un nombre de 0 à 8, par exemple radical -CH₂-CHCl₂, -CH₂-CHF₂ ou -CHF₂,
-(CH₂)ₙ-Hal dans lequel n et Hal sont définis comme ci-dessus, par exemple le radical -CH₂-CH₂Cl ou -CH₂-CH₂F,
-C-(C(Hal)₃)₃ dans lequel Hal est défini comme ci-dessus, par exemple un radical -C(CF₃)₃ ou 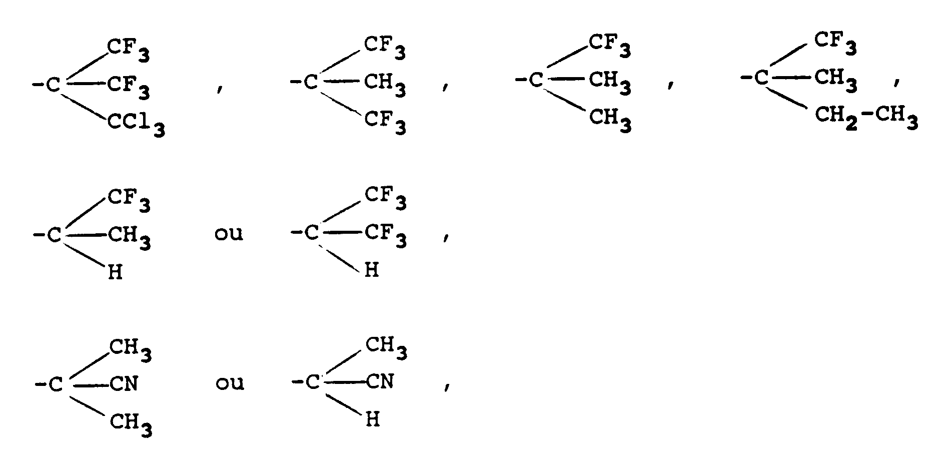
ou -(CH₂)ₙ-CN, dans lequel n est défini comme précédemment, 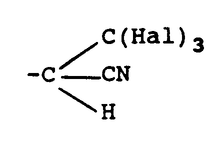
dans lequel Hal est défini comme précédemment, par exemple par le radical 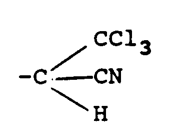
-(CH₂)ₙ-OR′, dans lequel n est défini comme précédemment et R′ représente un atome d'hydrogène ou un radical alkyle linéaire ou ramifié, comportant de 1 à 8 atomes de carbone, par exemple le radical -CH₂-OCH_{3'}, -CH₂-CH₂-O-CH_{3'}, -CH₂-CH₂-O-CH₂-CH₃ ou -CH₂-CH₂-OH, 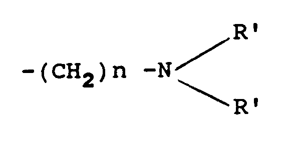
dans lequel n et R′ sont définis comme précédemment et les deux radicaux R′ peuvent être différents entre eux, par exemple le radical 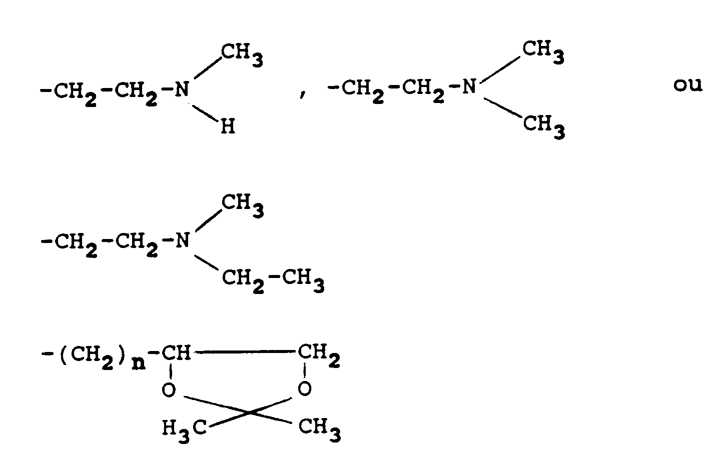
dans lequel n est défini comme précédemment, par exemple par le radical 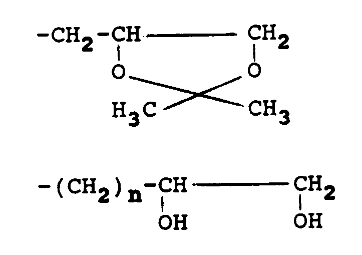
dans lequel n est défini comme précédemment, par exemple le radical 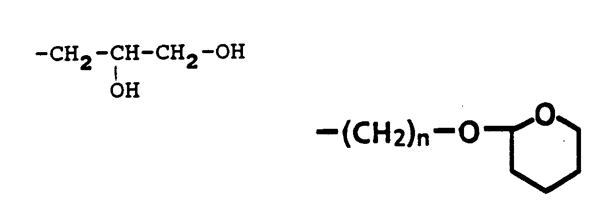
dans lequel n est défini comme précédemment, par exemple le radical 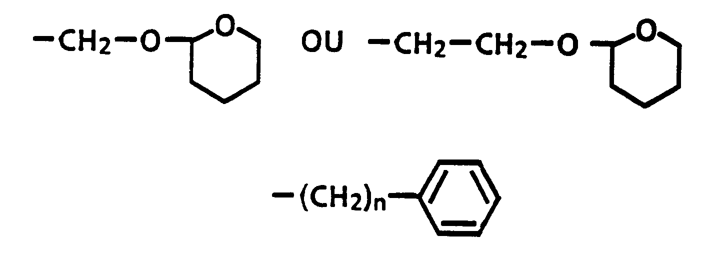
dans lequel n est défini comme précédemment, par exemple le radical benzyle ou phénéthyle, 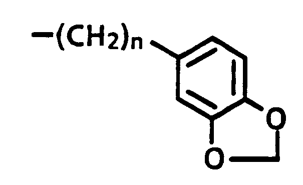
dans lequel n est défini comme précédemment, par exemple le radical 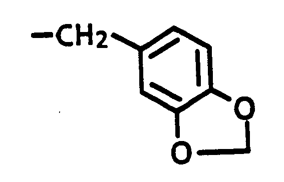
Lorsque R représente un radical aryle éventuellement substitué, il s'agit de préférence du radical phényle ou du radical phényle substitué par un ou plusieurs groupements OH, Oalc, alc renfermant de 1 à 8 atomes de carbone, ou par un halogène ou un groupement -CF₃, -OCF₃ ou SCF₃.

Lorsque R représente un radical hétérocyclique, il s'agit de préférence du radical pyridinyle, furanyle, thiophényle, oxazolyle ou thiazolyle.

L'invention a plus particulièrement pour objet les composés de formule (I) dans lesquels X représente un atome de fluor, les composés de formule (I) dans lesquels Z représente un atome d'hydrogène, les composés de formule (I) dans lesquels R représente un radical alkyle linéaire, ramifié, ou cyclique renfermant jusqu'à 4 atomes de carbone, notamment un radical éthyle.

Parmi les composés préférés de l'invention on peut citer plus particulièrement le composé de l'exemple 1.

Les composés de formule (I) présentent d'intéressantes propriétés qui permettent leur utilisation dans la lutte contre les parasites. Il peut s'agir par exemple de la lutte contre les parasites des locaux, les parasites des végétaux et les parasites des animaux à sang chaud.

C'est ainsi que l'on peut utiliser les produits de l'invention pour lutter contre les insectes, les nématodes et les acariens parasites des végétaux et des animaux.

L'invention a notamment pour objet l'application des composés de formule (I) à la lutte contre les parasites des locaux, les parasites des végétaux et les parasites des animaux à sang chaud.

Les produits de formule (I) peuvent être utilisés pour lutter contre les insectes dans les locaux, pour lutter notamment contre les mouches, les moustiques et les blattes.

Les produits de formule (I) peuvent également être utilisés également pour lutter contre les insectes dans le domaine agricole, pour lutter par exemple, contre les pucerons, les larves de lépidoptères et les coléoptères, ainsi que pour lutter contre les insectes du sol. Ils sont utilisés à des doses comprises entre 10 g et 300 g de matière active à l'hectare.

Les produits de formule (I) sont de plus photostables et ne sont pas toxiques pour les mammifères.

L'ensemble de ces propriétés fait des produits de formule (I) des produits qui correspondent parfaitement aux exigences de l'industrie agrochimique moderne : ils permettent de protéger les récoltes tout en préservant l'environnement.

Les produits de formule (I) peuvent aussi être utilisés pour lutter contre les acariens et les nématodes parasites des végétaux.

Les composés de formule (I) peuvent encore être utilisés pour lutter contre les acariens parasites des animaux, pour lutter par exemple contre les tiques et notamment les tiques de l'espèce Boophilus, ceux de l'espèce Hyalomnia, ceux de l'espèce Amblyomnia et ceux de l'espèce Rhipicephalus ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits de formule (I) définis ci-dessus.

L'invention a notamment pour objet les compositions insecticides renfermant comme principe actif au moins l'un des produits définis ci-dessus.

Ces compositions sont préparées selon les procédés usuels de l'industrie agrochimique ou de l'industrie vétérinaire ou de l'industrie des produits destinés à la nutrition animale.

Dans ces compositions destinées à l'usage agricole et à l'usage dans les locaux, la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un émanateur électrique.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudres, granulés, suspensions, émulsions, solutions.

Pour l'usage acaricide, on utilise de préférence des poudres mouillables pour pulvérisation foliaire, contenant de 1 à 80 % ou des liquides pour pulvérisation foliaire contenant de 1 à 500 g/l de principe actif. On peut également employer des poudres pour poudrage foliaire contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo [2,2-1] 5-heptène-2,3-dicarboximide, ou le pipéronyl-bis-2-(2′-n-butoxy éthoxy) éthylacétal (ou tropital).

Les composés de formule (I) présentent une excellente tolérance générale, et l'invention a donc également pour objet les produits de formule (I), pour lutter notamment contre les affections créées par les tiques et les gales chez l'homme et l'animal.

Les produits de l'invention sont notamment utilisés pour lutter contre les poux à titre préventif ou curatif et pour lutter contre la gale.

Les produits de l'invention peuvent être administrés par voie externe, par vaporisation, par shampooing, par bain ou badigeonnage.

Les produits de l'invention à usage vétérinaire peuvent être également administrés par badigeonnage de l'épine dorsale selon la méthode dite méthode "pour-on".

On peut indiquer également que les produits de l'invention peuvent être utilisés comme biocides ou comme régulateur de croissance.

L'invention a également pour objet les associations douées d'activité insecticide, acaricide ou nématicide, caractérisées en ce qu'elles contiennent comme matière active, d'une part, un au moins des composés de formule générale (I) et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropanecarboxyliques, par les esters d'alcool 3-phénoxy benzylique et d'alcools alpha-cyano 3-phénoxy benzyliques des acides 2,2-diméthyl 3-(2,2-dichlorovinyl) cyclopropanecarboxyliques, par les esters d'alcools alpha-cyano 3-phénoxy benzyliques d'acides 2,2-diméthyl 3-(2,2-dibromovinyl) cyclopropanecarboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimidométhylique, d'alcool 5-benzyl 3-furyl méthylique, d'alcool 3-phénoxy benzylique et d'alcool alpha-cyano 3-phénoxy benzylique des acides 2,2-diméthyl 3-(1,2,2,2-tétrahaloéthyl) cyclopropanecarboxyliques, dans lesquels "halo" représente un atome de fluor, de chlore ou de brome, étant entendu que les composés de formule (I) peuvent exister sous toutes leurs formes stéréoisomères possibles de même que les copules acides et alcools des esters pyréthrinoïdes ci-dessus.

L'invention a également pour objet un procédé de préparation des composés de formule (I) caractérisé en ce que l'on soumet un acide de formule (II) : 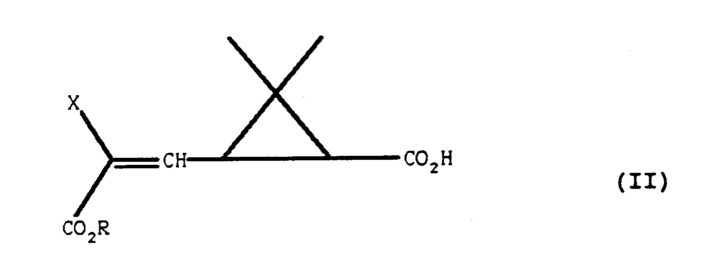
ou un dérivé fonctionnel de cet acide dans lequel X et R conservent leur signification précédente, à l'action d'un alcool de formule (III) : 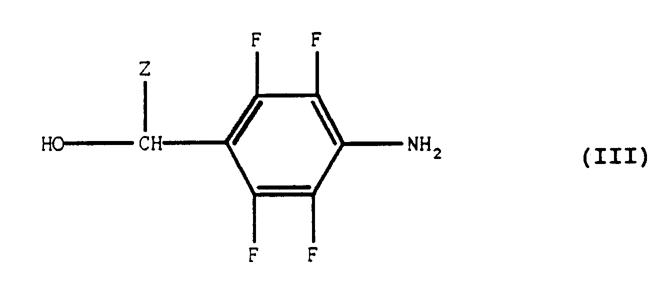
ou un dérivé fonctionnel de cet alcool dans lequel Z conserve sa signification précédente pour obtenir le composé de formule (I) correspondant.

Les composés de formule (II) sont des produits connus décrits dans les brevets européens 0038271, 0041021, 0048186, 0050534.

Les composés de formule (III) sont des composés connus d'une manière générale ; ils peuvent être préparés par exemple selon les procédés décrits dans le brevet EP 31199 ou encore Zh. Obshch Khim 37(6) 1300-6(1967).

### EXEMPLE 1 : [1R[1α,3α(ΔE)]] 2,2-diméthyl 3-[3-éthoxy 2-fluoro 3-oxopropényl] cyclopropannnecarboxylate de [2,3,5,6-tétrafluoro 4-amino phényl] méthyle.

On ajoute à 5°C, une solution renfermant 1,05 g de dicyclohexylcarbodiimide, 60 mg de diméthylaminopyridine et 10 cm³ de chlorure de méthylène, dans un mélange de 1 g d'alcool 4-amino 2,3,5,6-tétrafluorophénylméthylique (cf EP 31199 ou ZH Obshch Khim 37 (6), 1300-6 (1967) 40 cm³ de chlorure de méthylène et 1,29 g d'acide [1R[1α, 3α(ΔE)]] 2,2-diméthyl 3-[3-éthoxy 2-fluoro 3-oxopropényl] cyclopropanecarboxylique (préparé comme indiqué dans EP 0050534. On agite le mélange réactionnel pendant 4 heures à 20°C. On filtre, concentre et obtient 2,5 g d'un produit que l'on chromatographie sur silice en éluant avec le mélange hexane-acétate d'éthyle (8-2). On obtient 1,55 g du produit recherché.
[alpha]_{D} = 0 (c = 1 % CHCl₃).

### EXEMPLE 2 : [1R[1α,3α(ΔE)]] 2,2-diméthyl 3-[3-terbutyloxy 2-fluoro 3-oxopropényl] cyclopropanecarboxylate de [2,3,5,6-tétrafluoro 4-amino phényl] méthyle.

En opérant comme à l'exemple 1, à partir de l'alcool 4-amino 2,3,5,6-tétrafluorophénylméthylique et de l'acide [1R[1α,3α(ΔE)]] 2,2-diméthyl 3-[3-terbutyloxy 2-fluoro 3-oxopropényl] cyclopropanecarboxylique, on a obtenu le produit recherché F = 104°C.
[alpha]_{D} = +11,5° ± 1° (c = 0,95 % CHCl₃).

### EXEMPLE 3 : [1R[1α,3α(ΔZ)]] 2,2-diméthyl 3-[3-(1,1,1,3,3,3-hexafluoro) isopropoxy 3-oxopropényl] cyclopropanecarboxylate de [2,3,5,6-tétrafluoro 4-amino phényl] méthyle.

En opérant comme à l'exemple 1 à partir de 0,75 g d'alcool 4-amino 2,3,5,6-tétrafluorophényl méthylique et de 1,41 g d'acide [1R[1α,3α(ΔZ)] 2,2-diméthyl 3-[3-(1,1,1,3,3,3-hexafluoro) isopropoxy 3-oxopropényl] cyclopropane carboxylique, on a obtenu 2,33 g de produit attendu brut puis après chromatographie 1,1, g de produit pur.

### Spectre RMN :

1,29 (s), 1,33 (s) : CH₃ ; 2,02 (d), J=8,5 : H cyclopropyle ; 3,13 (m) : H₃ cyclopropyle ; 4,10 (s large) : NH₂ ; 5,13 (s) : CO₂-CH₂ ; 5,80 : CH-(CF₃)₂ ; 6,02 (d), J=11,5, 6,97 (dd), J=11 et 11,5 : H₂ et H₃ du propényl.

### EXEMPLE DE COMPOSITIONS

### Exemple de composition fumigène

On mélange d'une façon homogène :

| | |
|---|---|
| produit de l'exemple 1 | 0,25 g |
| poudre de tabu | 25,00 g |
| poudre de feuille de cèdre | 40,00 g |
| poudre de bois de pin | 33,75 g |
| vert brillant | 0,50 g |
| para-Nitrophénol | 0,50 g |

### ETUDE BIOLOGIQUE

### Etude de l'effet d'abattage sur mouche domestique

Les insectes tests sont des mouches domestiques femelles âgées de 4 jours. On opère par pulvérisation directe à la concentration de 0,25 g/l en chambre de Kearns et March en utilisant comme solvant un mélange d'acétone (5 %) et d'Isopar L (solvant pétrolier) (quantité de solvant utilisée 2 ml en une seconde). On utilise 50 insectes par traitement. On effectue les contrôles toutes les minutes jusqu'à 10 minutes, puis à 15 minutes et l'on détermine le KT 50 par les méthodes habituelles.

On a trouvé que la puissance relative du produit de l'exemple 1 par rapport à la bioallethrine était de 7,65.

## Revendications

1. Les composés de formule (I) : 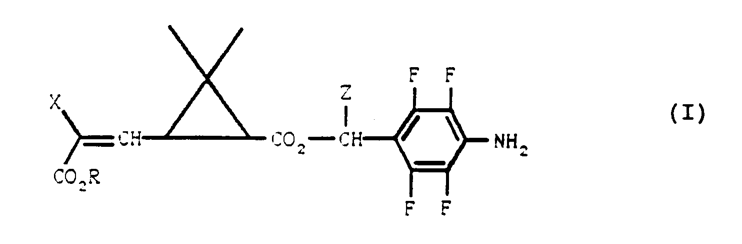 dans lesquels :
- X représente un atome d'hydrogène, de fluor, de chlore ou de brome,
- R représente un radical alkyle linéaire ramifié ou cyclique, saturé ou insaturé renfermant jusqu'à 8 atomes de carbone éventuellement substitué, un radical aryle renfermant jusqu'à 14 atomes de carbone éventuellement substitué ou un radical hétérocyclique éventuellement substitué,
- Z représente un atome d'hydrogène, un radical CH₃, C≡N ou C≡CH, sous toutes leurs formes stéréoisomères possibles ainsi que leurs mélanges.

2. Les composés de formule (I) tels que définis à la revendication 1 dans lesquels X représente un atome de fluor.

3. Les composés de formule (I) tels que définis à l'une quelconque des revendications 1 et 2 dans lesquels Z représente un atome d'hydrogène.

4. Les composés de formule (I) tels que définis à l'une quelconque des revendications 1 à 3 dans lesquels R représente un radical alkyle linéaire, ramifié, ou cyclique renfermant jusqu'à 4 atomes de carbone.

5. Les composés de formule (I) tels que définis à la revendication 4 dans lesquels R représente un radical éthyle.

6. Le [1R[1α,3α(ΔE)]] 2,2-diméthyl 3-[3-éthoxy 2-fluoro 3-oxopropenyl] cyclopropanecarboxylate de [2,3,5,6-tétrafluoro 4-amino phényl] méthyle.

7. Les compositions pesticides destinées à la lutte contre les parasites des végétaux, les parasites des locaux et les parasites des animaux à sang chaud, caractérisées en ce qu'elles renferment comme principe actif au moins un composé de formule (I) tel que défini à l'une quelconque des revendications 1 à 5.

8. Les compositions pesticides destinées à la lutte contre les parasites des végétaux, les parasites des locaux et les parasites des animaux à sang chaud, caractérisées en ce qu'elles renferment comme principe actif le composé défini à la revendication 6.

9. Les compositions insecticides renfermant comme principe actif au moins un composé de formule (I) tel que défini à l'une quelconque des revendications 1 à 6.

10. Les compositions acaricides renfermant comme principe actif au moins un composé de formule (I) tel que défini à l'une quelconque des revendications 1 à 6.

11. Les compositions nématicides renfermant comme principe actif au moins un composé de formule (I) tel que défini à l'une quelconque des revendications 1 à 6.

12. Les compositions douées d'activité insecticide, acaricide ou nématicide, caractérisées en ce qu'elles contiennent comme matière active, d'une part, un au moins des composés de formule générale (I) et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropanecarboxyliques, par les esters d'alcool 3-phénoxy benzylique et d'alcools alpha-cyano 3-phénoxy benzyliques des acides 2,2-diméthyl 3-(2,2-dichlorovinyl) cyclopropanecarboxyliques, par les esters d'alcools alpha-cyano 3-phénoxy benzyliques d'acides 2,2-diméthyl 3-(2,2-dibromovinyl) cyclopropanecarboxyliques, par les esters d'alcool 3-phénoxy benzylique des acides 2-parachlorophènyl 2-isopropyl acétiques, par les esters d'alléthrolones, d'alcool 3,4,5,6-tétrahydrophtalimidométhylique, d'alcool 5-benzyl 3-furyl méthylique, d'alcool 3-phénoxy benzylique et d'alcool alpha-cyano 3-phénoxy benzylique des acides 2,2-diméthyl 3-(1,2,2,2-tétrahaloéthyl) cyclopropanecarboxyliques, dans lesquels "halo" représente un atome de fluor, de chlore ou de brome, étant entendu que les composés de formule (I) peuvent exister sous toutes leurs formes stéréoisomères possibles de même que les copules acides et alcools des esters pyréthrinoïdes ci-dessus.

13. Procédé de préparation des composés de formule (I) tels que définis à l'une quelconque des revendications 1 à 6 caractérisé en ce que l'on soumet un acide de formule (II) : 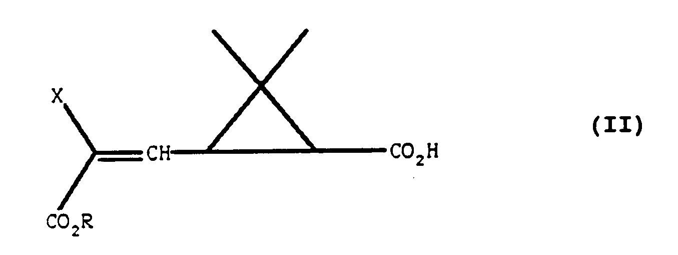 ou un dérivé fonctionnel de cet acide avec un alcool de formule (III) : 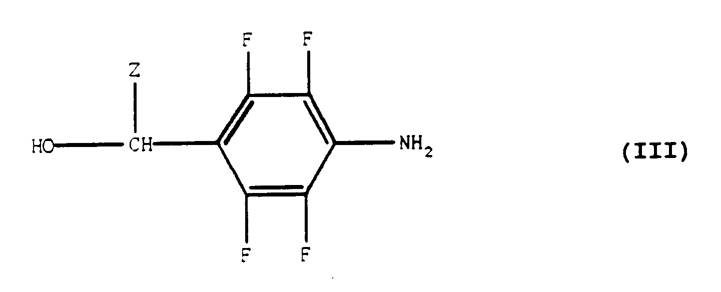 pour obtenir le composé de formule (I) correspondant.

## Claims

1. The compounds of formula (I): 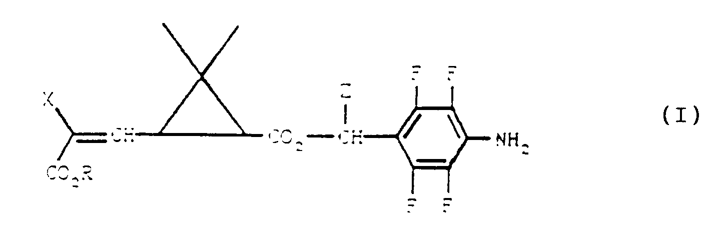 in which:
- X represents a hydrogen, fluorine, chlorine or bromine atom,
- R represents an optionally substituted, saturated or unsaturated, linear, branched or cyclic alkyl radical containing up to 8 carbon atoms, an optionally substituted aryl radical containing up to 14 carbon atoms or an optionally substituted heterocyclic radical,
- Z represents a hydrogen atom, a CH₃, C≡N or C≡CH radical, in all their possible stereoisomeric forms as well as their mixtures.

2. The compounds of formula (I) as defined in claim 1 in which X represents a fluorine atom.

3. The compounds of formula (I) as defined in any one of claims 1 and 2 in which Z represents a hydrogen atom.

4. The compounds of formula (I) as defined in any one of claims 1 to 3 in which R represents a linear, branched or cyclic alkyl radical containing up to 4 carbon atoms.

5. The compounds of formula (I) as defined in claim 4 in which R represents an ethyl radical.

6. [2,3,5,6-tetrafluoro 4-amino phenyl] methyl [1R [1α,3α (ΔE)]] 2,2-dimethyl 3-[3-ethoxy 2-fluoro 3-oxopropenyl] cyclopropanecarboxylate.

7. The pesticide compositions intended for combating parasites of vegetation, parasites of premises and parasites of warm-blooded animals, characterized in that they contain as active ingredient at least one compound of formula (I) as defined in any one of claims 1 to 5.

8. The pesticide compositions intended for combating parasites of vegetation, parasites of premises and parasites of warm-blooded animals, characterized in that they contain as active ingredient the compound defined in claim 6.

9. The insecticide compositions containing as active ingredient at least one compound of formula (I) as defined in any one of claims 1 to 6.

10. The acaricide compositions containing as active ingredient at least one compound of formula (I) as defined in any one of claims 1 to 6.

11. The nematicide compositions containing as active ingredient at least one compound of formula (I) as defined in any one of claims 1 to 6.

12. The compositions endowed with insecticide, acaricide or nematicide activity, characterized in that they contain as active ingredient, on the one hand at least one of the compounds of general formula (I) and on the other hand at least one of the pyrethrinoid esters chosen from the group constituted by the esters of allethrolones, of 3,4,5,6-tetrahydrophthal-imidomethyl alcohol, of 5-benzyl-3-furyl methyl alcohol, of 3-phenoxybenzyl alcohol and of alpha-cyano-3-phenoxybenzyl alcohols with chrysanthemic acids, by the esters of 5-benzyl-3-furyl methyl alcohol with 2,2-dimethyl-3-(2-oxo-3-tetrahydrothiophenylidene methyl)-cyclopropanecarboxylic acids, by the esters of 3-phenoxybenzyl alcohol and of alpha-cyano-3-phenoxybenzyl alcohols with 2,2-dimethyl-3-(2,2-dichlorovinyl)-cyclopropanecarboxylic acids, by the esters of alpha-cyano-3-phenoxy-benzyl alcohols with 2,2-dimethyl-3-(2,2-dibromovinyl)-cyclopropanecarboxylic acids, by the esters of 3-phenoxybenzyl alcohol with 2-parachlorophenyl-2-isopropyl acetic acids, by the esters of allethrolones, of 3,4,5,6-tetrahydrophthalimidomethyl alcohol, of 5-benzyl-3-furyl methyl alcohol, of 3-phenoxybenzyl alcohol and of alpha-cyano-3-phenoxybenzyl alcohol with 2,2-dimethyl-3-(1,2,2,2-tetrahaloethyl)-cyclopropanecarboxylic acids, in which "halo" represents a fluorine, chlorine or bromine atom, it being understood that the compounds of formula (I) can exist in all their possible stereoisomeric forms, as well as the acid and alcohol copulas of the above pyrethrinoid esters.

13. Preparation process for the compounds of formula (I) as defined in any one of claims 1 to 6, characterized in that an acid of formula (II): 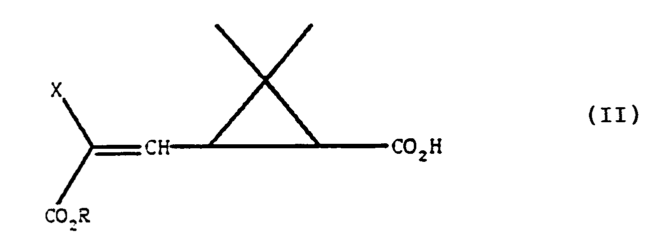 or a functional derivative of this acid, is reacted with an alcohol of formula (III): 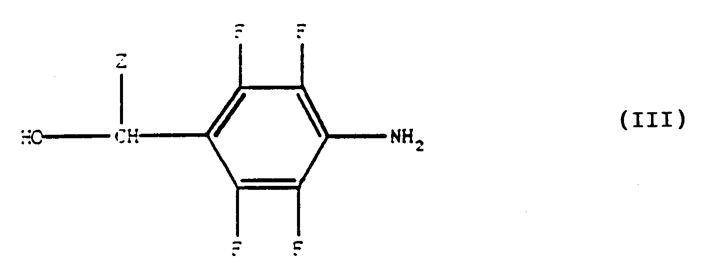 in order to obtain the corresponding compound of formula (I).

## Patentansprüche

1. Verbindungen der Formel (I) 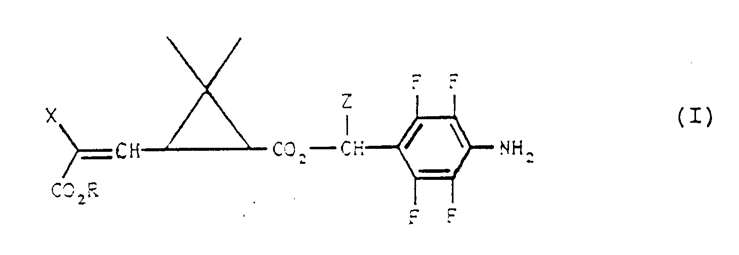 worin
X für ein Wasserstoff-, Fluor-, Chlor- oder Bromatom steht,
R einen gesättigten oder ungesättigten, linearen, verzweigten oder cyclischen Alkylrest mit bis zu 8 Kohlenstoffatomen, der gegebenenfalls substituiert ist, einen Arylrest mit bis zu 14 Kohlenstoffatomen, der gegebenenfalls substituiert ist, oder einen gegebenenfalls substituierten heterocyclischen Rest bedeutet,
Z für ein Wasserstoffatom, einen Rest CH₃, C≡N oder C≡CH steht, in sämtlichen ihrer möglichen stereoisomeren Formen sowie deren Gemische.

2. Verbindungen der Formel (I) gemäß Anspruch 1,
worin X für ein Fluoratom steht.

3. Verbindungen der Formel (I) gemäß Anspruch 1 und 2, worin Z für ein Wasserstoffatom steht.

4. Verbindungen der Formel (I) gemäß einem der Ansprüche 1 bis 3, worin R für einen linearen, verzweigten oder cyclischen Alkylrest mit bis zu 4 Kohlenstoffatomen steht.

5. Verbindungen der Formel (I) gemäß Anspruch 4, worin R einen Ethylrest bedeutet.

6. [1R-[1α,3α(ΔE)]]-2,2-Dimethyl-3-[3-ethoxy-2-fluor-3-oxopropenyl]-cyclopropancarbonsäure-(2,3,5,6-tetrafluor-4-aminophenyl)-methylester.

7. Pestizide Zusammensetzungen für die Bekämpfung der Parasiten von Pflanzen, Parasiten von Räumlichkeiten und Parasiten warmblütiger Tiere, dadurch gekennzeichnet, daß sie als Wirkstoff zumindest eine Verbindung der Formel (I), wie in einem der Ansprüche 1 bis 5 definiert, enthalten.

8. Pestizide Zusammensetzungen für die Bekämpfung von Parasiten der Pflanzen, Parasiten von Räumlichkeiten und Parasiten warmblütiger Tiere, dadurch gekennzeichnet, daß sie als Wirkstoff die in Anspruch 6 definierte Verbindung enthalten.

9. Insektizide Zusammensetzungen, enthaltend als Wirkstoff zumindest eine Verbindung der Formel (I), wie in einem der Ansprüche 1 bis 6 definiert.

10. Akarizide Zusammensetzungen, enthaltend als Wirkstoff zumindest eine Verbindung der Formel (I), wie in einem der Ansprüche 1 bis 6 definiert.

11. Nematizide Zusammensetzungen, enthaltend als Wirkstoff zumindest eine Verbindung der Formel (I), wie in einem der Ansprüche 1 bis 6 definiert.

12. Zusammensetzungen mit insektizider, akarizider oder nematizider Aktivität, dadurch gekennzeichnet, daß sie als Wirkstoff einesteils zumindest eine der Verbindungen der allgemeinen Formel (I) und anderenteils zumindest einen Ester der Pyrethrinoide, ausgewählt unter den Estern der Allethrolone, des 3,4,5,6-Tetrahydrophthalimido-methylalkohols, des 5-Benzyl-3-furyl-methylalkohols, des 3-Phenoxybenzylalkohols und der α-Cyano-3-phenoxy-benzylalkohole der Chrysanthemumsäuren, unter den Estern des 5-Benzyl-3-furyl-methylalkohols der 2,2-Dimethyl-3-(2-oxo-3-tetrahydrothiophenyliden-methyl)-cyclopropancarbonsäuren, unter den Estern des 3-Phenoxybenzylalkohols und der α-Cyano-3-phenoxy-benzylalkohole der 2,2-Dimethyl-3-(2,2-dichlorvinyl)-cyclopropancarbonsäuren, unter den Estern der α-Cyano-3-phenoxy-benzylalkohole, der 2,2-Dimethyl-3-(2,2-dibromvinyl)-cyclopropancarbonsäuren, unter den Estern des 3-Phenoxybenzylalkohols der 2-para-Chlorphenyl-2-isopropylessigsäuren, unter den Estern der Allethrolone, des 3,4, 5,6-Tetrahydrophthalimido-methylalkohols, des 5-Benzyl-3-furyl-methylalkohols, des 3-Phenoxybenzylalkohols und des α-Cyano-3-phenoxy-benzylalkohols der 2,2-Dimethyl-3-(1,2, 2,2-tetrahaloethyl)-cyclopropancarbonsäuren, worin "halo" für ein Fluor-, Chlor- oder Bromatom steht, enthalten, mit der Maßgabe, daß die Verbindungen der Formel (I) in sämtlichen ihrer möglichen stereoisomeren Formen ebenso wie die Säure- und Alkoholverknüpfungskomponenten der vorstehenden Pyrethrinoidester vorliegen können.

13. Verfahren zur Herstellung der Verbindungen der Formel (I), wie in einem der Ansprüche 1 bis 6 definiert, dadurch gekennzeichnet, daß man eine Säure der Formel (II) 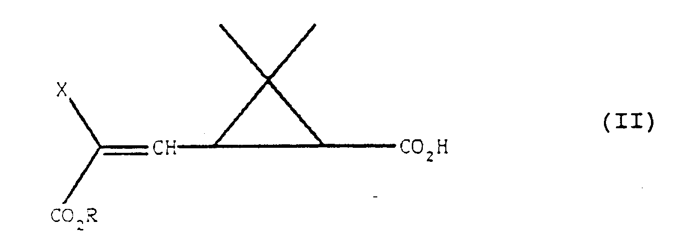 oder ein funktionelles Derivat dieser Säure mit einem Alkohol der Formel (III) 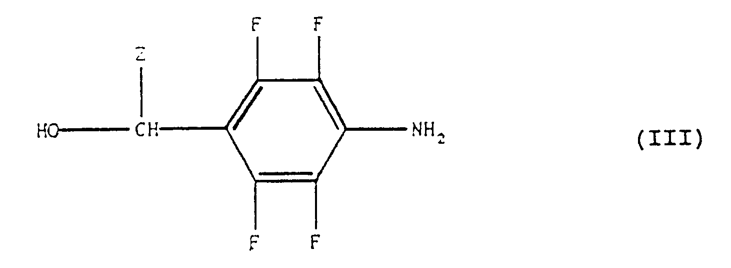 umsetzt, um zu der entsprechenden Verbindung der Formel (I) zu gelangen.
